# EUROPEAN PATENT APPLICATION

(11) **EP 0 661 055 A1**
(43) Date of publication of application: **05.07.1995**
(21) Application number: 94203756.5
(22) Date of filing: 23.12.1994
(51) Int. Cl.: A61K 31/71

(54) **Pharmaceutical formulation containing clobetasone and tobramycin and applications thereof**

(30) Priority: 31.12.1993 ES 9302735
(71) Applicant: LABORATORIOS CUSI, S.A., E-08320 El Masnou (Barcelona) (ES)
(72) Inventor: Bergamini, Michael Van Wie, 08328 Alella (Barcelona) (ES); Lopez Cabrera, Antonio, E-08018 Barcelona (ES); Vallet Mas, José Alberto, E-08022 Balmes (Barcelona) (ES); Oros Laguens, Carmen, E-08029 Barcelona (ES)
(74) Representative: Ungria Lopez, Javier

(57) **Abstract**

The composition comprises: 0.001-5.0 % Clobetasone or an isomer or a derivative or one of the pharmaceutically acceptable salts thereof; 0.05-5.0 % Tobramycin or an isomer or a derivative or one of the pharmaceutically acceptable salts thereof; and, optionally, one or more ingredients selected from among isotonizing agents, pH buffers, viscosity modifying agents, wetting agents, chelating agents, antioxidants, preservatives, solubility agents, aside from one or several excipients suitable to the pharmaceutical form for application of the formulation. Said formulation has a pH between 4 and 9.

Application in the treatment of eye and ear processes accompanied by infection.

## Description

### TECHNICAL FIELD OF THE INVENTION

The present invention fits in the technical field of formulations used to treat inflammatory eye and ear processes that are accompanied by infection.

Specifically, the present invention provides a formulation comprised of Clobetasone and Tobramycin for ophthalmic and otic topical use.

### PRIOR ART

The combinations of an anti-inflammatory drug and an antibiotic in general, and of an anti-inflammatory drug and Tobramycin specifically, are known and used in treatment of eye and ear pathologies. However, in most cases the anti-inflammatory drug is a strong steroid that can have important adverse effects. Specifically, in ophthalmology, an important increase of the intra-ocular pressure and/or a predisposition to infections as a result of the immunosuppressor effect attributable to the steroid anti-inflammatory drug can be observed.

The present invention provides a formulation comprised of Clobetasone and Tobramycin for topical use in inflammatory eye and ear processes that are accompanied by infection. Said formulation minimizes the adverse effects associated with the use of strong sterioid anti-inflammatory drugs.

### DETAILED DESCRIPTION OF THE INVENTION

The present invention refers to the combination of a steroid anti-inflammatory drug, Clobetasone, and an antibiotic of the family of aminoglycosides, Tobramycin, for topical use thereof in inflammatory eye and ear processes that are accompanied by infection.

The pathological eye processes that can be treated with the formulations described in the present invention are conjunctivitis or any eye traumatism due to injury or surgery. Besides, eye inflammations and infections can also be treated with the formulations described in the present invention.

Likewise, with the formulations described in the present invention, different pathological eye processes, such as otitis externa, for example, can be treated. Besides, ear inflammations and infections can also be treated with the formulations described in the present invention.

Clobetasone is a steroid anti-inflammatory drug that is chemically known as (16-beta)-21-chloro-fluoro-17-hydroxy-16-methylpregna-1,4-diene-3,11,20-trione. Clobetasone has the structural formula (I): 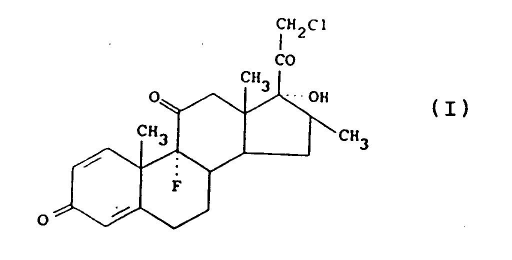
Tobramycin is an antibacterial agent belonging to the aminoglycoside family, water soluble and known chemically as 4-[2,6-diamino-2,3,6-trideoxy-alpha-D-glycopyranosyl]-6-[3-amino-3-deoxy-alpha-D-glycopyranosyl]-2-deoxystreptamine. Tobramycin has the structural formula (II): 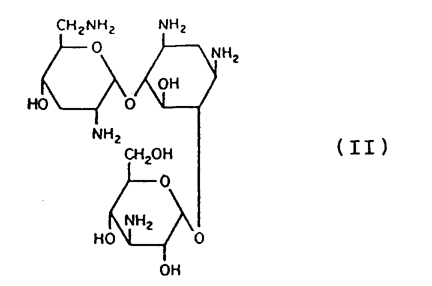
Tobramycin has a broad action spectrum against Gram positive as well as Gram negative organisms. The main susceptible microorganisms are S. aureus, S. epidermidis, Stretococcus pneumoniae, Pseudomonas aeruginosa, Escherichia coli, Enterobacter aerogenes, Proteus mirabilis, Klebsiella pneumoniae, Morganella morganii, Haemophilius influenzae, Haemophilius aegyptius, Moraxela lacunata and Acinetobacter calcoaceticus.

Likewise, the present invention includes the isomers, derivatives and pharmaceutically acceptable salts of Clobetasone and of Tobramycin.

The compositions of the present invention comprise the combination of a steroid anti-inflammatory drug, Clobetasone, and an antibiotic, Tobramycin. The compositions also include other compounds traditionally used in ophthalmic and otic preparations, such as isotonizing agents, pH buffers, viscosity modifying agents, wetting agents, chelating agents, antioxidants and preservatives.

Including any representative of the above mentioned groups of compounds depends on the characteristics that the final preparations are to have. The choice of one compound or the other depends on the physical, physico-chemical and chemical characteristics of the rest of the components of the formulation in order to obtain a stable, well tolerated and therapeutically effective preparation.

In the following paragraphs, in the descriptive part of the present specification as well as in the set of claims, the percentages are expressed by weight/volume in those cases in which the resulting pharmaceutical form is liquid. In other words, a solution, suspension, emulsion, etc. However, in the cases in which the resulting product of the invention is applied as a solid or semi-solid, such as an ointment, gel, etc., the percentage is expressed by weight/weight.

Sodium chloride, sodium sulfate, glycerol, mannitol and sorbitol, among others, can be cited as isotonizing agents. These compounds are used to achieve the required tonicity in the preparation. These compounds are typically used at some levels between 0.4 and 3.0 %.

Acetates, citrates, borates, phosphates, tris(hydroxymethyl)-aminomethane and amino acids, such as glycine, lysin, glutamic acid, arginine and aspartic acid, among others, can be given as pH buffers. This type of product is included in formulations to keep the pH stable during the life of the product and to improve tolerance when the use of the product so requires this. These compounds are typically used at some levels between 0.01 and 3.0 %.

As viscosity modifying agents, that improve the time during which the product remains where it has been administered or that ensure the homogenity of the dosage in the cases in which the resulting product is a suspension, polyvinyl alcohol, polyvinylpyrrolidone, methylcellulose, hydroxypropylcellulose, hydroxyethylcellulose, carboxymethylcellulose, hydroxypropylmethylcellulose, among others, can be cited. These compounds are typically used at some levels between 0.01 and 10.0 %.

As wetting agents, that improve the contact between the solid and the aqueous vehicle in the compositions that turn out to be suspensions or that improve solubility of some of the components of the formulation, polyethoxylated fatty alcohols, polyethoxylated alkylphenols, polyethoxylated fatty acids, sorbitan esters, alkaneamides, cetyl alcohol, glycerol-polyethyleneglycol esters and fatty acids or mixtures of the cited components, among other non-ionic surface active agents can be cited. These compounds are typically used at some levels between 0.01 and 7.0%.

As chelating agents we can cite citric acid and disodium salt of ethylenediaminetetraacetic acid, EDTA. These compounds are included to eliminate the heavy metals from the solution and to improve the action of the preservatives. These compounds are typically used at levels between 0.01 and 2.0 %.

As antioxidants, to stabilize the formulations included in the present invention, we can emphasize ascorbic acid, BHT, BHA, sodium sulfite, sodium bisulfite and sodium metabisulfite, among others. These compounds are typically used at some levels between 0.01 and 2.0%.

As preservatives, to prevent contamination of the product, quaternary ammonium derivatives, such as benzalkonium chloride, cetylmethylammonium bromide, cetylpyridine chloride, benzethonium chloride, organomercury derivatives such as Thimerosal, phenylmercury acetate and phenylmercury nitrate, methyl and propyl p-hydroxybenzoates and sodium salsts thereof, Chlorobutanol, beta-phenylethyl alcohol, benzyl alcohol, chlorhexidine salts such as chlorhexidine diacetate, digluconate, sorbic acid, cetrimide, phenylethyl alcohol and phenoxyethanol, or a mixture of the cited compounds, among others, can be cited. The formulations of the present invention also include the mixtures of the cited compounds. These compounds are typically used at levels between 0.005 and 1.0 %.

Other optional excipients, that may be used in terms of the final characteristics that the preparation is to have, may be agents that increase solubility (cyclodextrine, polyethylene glycols, propylene glycols, dextrane), the excipients normally used in pharmaceutical ointments (mineral oil, paraffin, lanolin, cholesterol, etc.) or the excipients normally used to obtain pharmaceutical hydrogels (poly(hydroxyethylmethacrylate), poly(N-vinylpyrrolidone), polyvinyl alcohol, acrylic acid polymers such as Carbopol, etc.). These compounds are typically used at some levels between 0.01 and 25.0%.

Aside from the elaboration of the formulations included in the present invention excipients that allow some of the components of the same to be microencapsulated can also be used. Hence, the following can be used:
- Lipid substances such as coconut oil derivatives, ethoxylated oleic glycerides, diethylene glycol monoethyl ether, C₈-C₁₀ ethoxylated glycerides, phospholipids, natural oils or petroleum derivatives or a mixture of several of them. These substances are typically used at some levels between 0.01 and 20.0%.
- Viscosity modifying agents, such as the ones cited above in the present document.
- Biocompatible polymers, such as a polyacrylic, polylactic, polyglycolic derivative, a polylacticglycolic copolymer, a polyanhydride, a polyamide, a poly (alpha, amino acid), cellulosic polymers, natural polymers, or a mixture of two or more of the cited compounds. These compounds are typically used at some levels between 0.01 and 5.0%.

The present invention includes the different pharmaceutical forms (solutions, suspensions, emulsions, ointments or hydrogels) obtained upon combining the different above mentioned compounds. The characteristics of the components chosen can condition the pharmaceutical form needed to obtain a stable, well tolerated and therapeutically effective preparation.

In accordance with the present invention, the resulting combination includes between 0.001 and 5% Clobetasone or a pharmaceutically acceptable derivative thereof, preferably between 0.05 and 3% Clobetasone or a pharmaceutically acceptable derivative thereof and between 0.05 and 5.0 % Tobramycin or a pharmaceutically acceptable derivative thereof, preferably between 0.10 and 3.0 % of Tobramycin or a pharmaceutically acceptable derivative thereof.

The pH of the formulations included in the present invention vary between 4 and 9. To adjust the pH of the formulations to the desired value, aside from the above cited pH buffers, acids (hydrochloric acid, sulfuric acid, etc.) or bases (sodium hydroxide, potassium hydroxide, etc.) can be used.

The amount of the preparation that can be administered to the receptor animal depends on the nature thereof (species, age, size) as well as the general state of health and on the severity and type of disease the animal has. Although the doctor has to establish the dosage, it is recommended that the formulations included in the present invention be administered 2 to 4 times a day, instilling one or two drops each time.

The formulations included in the present invention can be packaged in the containers normally used for this type of preparation, in accordance with the type of resulting pharmaceutical form.

In the cases in which use thereof so requires, the combinations included in the present invention can be prepared in sterile conditions.

The presence of Tobramycin, or of a pharmaceutically acceptable derivative thereof does not affect the activity of the anti-inflammatory drug used, just like the presence of Clobetasone, or of a pharmaceutically acceptable derivative thereof, does not interfere in the antimicrobial activity of the antibiotic.

Using the suitable combination of the components described in the present invention formulations that turn out from an antimicrobial point of view to be effective according to the criteria of the European Pharmacopeia are obtained.

### EMBODIMENTS OF THE INVENTION

The following formulations are given as representative examples of the compositions included in the present invention and should not be considered as restrictions of the scope of the present invention.

### EXAMPLE N^{º} 1. Ophthalmic suspension

| Substance | Amount per 100 ml. |
|---|---|
| Clobetasone 17 butyrate | 0.100 g |
| Tobramycin | 0.300 g |
| Hydroxypropylmethylcellulose | 0.500 g |
| Glycerol q.s. | 300 mOsmol/kg |
| Polysorbate 80 | 0.100 g |
| EDTA Na₂ | 0.025 g |
| Benzalkonium chloride | 0.005 g |
| Hydrochloric acid/sodium hydroxide q.s. | pH 4.8 - 5.6 |
| Purified water q.s. | 100 ml |

In order to obtain the ophthalmic suspension, 90 % of the water of the formulation is placed in a suitable container and the benzalkonium chloride, glycerol, EDTA and Tobramycin are added. The pH is adjusted with hydrochloric acid or sodium hydroxide and the following components are added: Polysorbate 80 and hydroxypropylmethylcellulose. The volume is completed with purified water and the resulting solution is filtered through a previously sterilized 0.22 micra filtration system. The resulting filtrate is collected in a sterile area and the Clobetasone 17 Butyrate, previously sterilized, is added in these conditions. The system is homogenized with a rotary agitator. The obtained suspension is dosed in suitable previously sterilized containers.

### EXAMPLE N^{º} 2. Ophthalmic suspension

| Substance | Amount per 100 ml. |
|---|---|
| Clobetasone 17 Butyrate | 0.100 g |
| Tobramycin | 0.300 g |
| Hydroxyethylcellulose | 0.250 g |
| Sodium chloride q.s. | 300 mOsmol/kg |
| Polysorbate 80 | 0.100 g |
| EDTA Na₂ | 0.100 g |
| Sorbic acid | 0.200 g |
| Hydrochloric acid/sodium hydroxide q.s. | pH 4.8 - 5.6 |
| Purified water q.s. | 100 ml |

To obtain the ophthalmic suspension, 90 % of the water of the formulation is placed in a suitable container, and the sorbic acid, sodium chloride, EDTA and Tobramycin are added. The pH is adjusted with hydrochloric acid or sodium hydroxide and the following components are added: Polysorbate 80 and hydroxyethylcellulose. The volume is completed with purified water and the resulting solution is filtered through a previously sterilized 0.22 micra filtration system. The resulting filtrate is collected in a sterile area and the previously sterilized Clobetasone is added in these conditions. The system is homogenized with a rotary agitator. The obtained suspension is dosed in previously sterilized suitable containers.

### EXAMPLE N^{º} 3. Ophthalmic ointment

| Substance | Amount per 100 g |
|---|---|
| Clobetasone 17 butyrate | 0.100 g |
| Tobramycin | 0.300 g |
| Paraffin q.s. | 100 g |

To obtain the ophthalmic ointment, each one of the active principles, previously sterilized, are mixed separately with an amount of paraffin 50 times higher than the weight thereof. Each one of the mixtures is refined separately through a roll grinder and then they are mixed with the remaining amount of paraffin. The mixture is homogenized in a beater, it is refined again in a roll grinder if necessary and finally, it is dosed in previously sterilized tubes.

### EXAMPLE N^{º} 4. Suspension with nanocapsules

| Substance | Amount per 100 ml |
|---|---|
| Clobetasone 17 Butyrate | 0.100 g |
| Tobramycin | 0.300 g |
| Poloxamer 188 | 2.500 g |
| Polylactic-glycolic 75:25 | 1.250 g |
| Phosphatidyl choline 95% | 2.500 g |
| C₈-C₁₂saturated fatty acid triglycerides | 5.000 g |
| Benzalkonium chloride | 0.02 g |
| Purified water q.s. | 100.0 ml |

- Aqueous Phase: The Poloxamer 188 is dissolved in an amount of water corresponding to 5 times the final volume of the formulation and it is filtered through a 0.22 µm filter.
- Organic Phase I: The polylactic-glycolic copolymer (75:25) and the phosphatidyl choline 95 % are dissolved in the necessary amount of acetone.
- Organic Phase II: The Clobetasone 17-butyrate is dissolved in the C₈-C₁₂ saturated fatty acid triglycerides.
- Organic Phase III: Organic phase II is added to Organic Phase I and it is completed with acetone until equal to 2.5 times the final volume of the formula is obtained. The Organic Phase III is added slowly to the aqueous one until an Organic Phase III:Aqueous Phase ratio of 1:2 is reached. The two phase are continued to be added simultaneously to the colloidal suspension, removing the same as it is formed and the organic solvent is eliminated under a vacuum until 90% of the final volume of the formula is reached. The benzalkonium chloride and Tobramycin are added, the pH is adjusted to a value in a range between 4.8 - 5.6 and the final volume of the formula is completed with water.

## Claims

1. Pharmaceutical formulation containing Clobetasone and Tobramycin for its ophthalmic and otic topical use, characterized in that it has a pH between 4 and 9 and in that it comprises:
- 0.001-5.0% of Clobetasone or an isomer, or a derivative or one of the pharmaceutically acceptable salts thereof;
- 0.05-5.0% of Tobramycin or an isomer, or a derivative or one of the pharmaceutically acceptable salts thereof;
- optionally, 0.4-3.0% of an isotonizing agents;
- optionally, 0.01-3.0% of a pH buffer;
- optionally, 0.01-10.0% of a viscosity modifying agent;
- optionally, 0.01-7.0% of a wetting agent;
- optionally, 0.01-2.0% of a chelating agent;
- optionally, 0.01-2.0% of an antioxidant;
- optionally, 0.0005-1.0% of a preservative;
- optionally, 0.01-25.0% of an agent that increases solubility;
- optionally, 0.01-25.0% of an excipient used in pharmaceutical ointments;
- optionally, 0.01-25.0% of an excipient used in pharmaceuticaly hydrogels;
- optionally, 0.01-20.0% of lipid substances that take part in the microencapsulation of the components of the formulation;
- optionally, 0.01-5.0% of biocompatible polymers that take part in the microencapsulation of the components of the formulation.

2. A formulation, according to claim 1, characterized in that the isotonizing agent is selected from the group formed by sodium chloride, sodium sulfate, glycerol, mannitol and sorbitol.

3. A formulation according to claim 1, characterized in that the pH buffer is selected from the group formed by acetate, citrates, borates, phosphates, tris (hydroxymethyl)aminomethane and amino acids such as glycine, lysin, glutamic acid, arginine and aspartic acid.

4. A formulation according to claim 1, characterized in that the viscosity modifying agent is selected from the group formed by polyvinyl alcohol, polyvinylpyrrolidone, methylcellulose, hydroxypropylcellulose, hydroxyethylcellulose, carboxymethylcellulose and hydroxypropylmethylcellulose.

5. A formulation, according to claim 1, characterized in that the wetting agent is selected from the group formed by polyethoxylated fatty alcohols, polyethoxylated alkylphenols, polyethoxylated fatty acids, sorbitan esters, alkaneamides, cetyl alcohol, glycerol-polyethyleneglycol esters and fatty acids, polyethyleneglycol esters and fatty acids or mixtures of the cited components.

6. A formulation , according to claim 1, characterized in that the chelating agent is selected from the group formed by citric acid and disodium salt of ethylenediaminetetraacetic acid, EDTA.

7. A formulation, according to claim 1, characterized in that the antioxidant is selected from the group formed by ascorbic acid, BHT, BHA, sodium sulfite, sodium bisulfite and sodium metabisulfite.

8. A formulation, according to claim 1, characterized in that the preservative is selected from the group formed by quaternary ammonium derivatives such as benzalkonium chloride, cetylmethylammonium bromide, cetylpyridine chloride, benzethonium chloride, organomercury derivatives such as Thimerosal, phenylmercury acetate and phenylmercury acetate and methyl and propyl p-hydroxybenzoates and sodium salts thereof, Chlorobutanol, beta-phenylethyl alcohol, benzyl alcohol, chlorhexidine salts such as chlorhexidine diacetate, digluconate, sorbic acid, cetrimide, phenylethyl alcohol and phenoxyethanol, or a mixture of the cited compounds.

9. A formulation, according to claim 1, characteized in that the excipient is selected from the agents that increase solubility (cyclodextrines, polyethylene glycols, propylene glycols, dextranes), the excipients normally used in pharmaceutical ointments (mineral oil, paraffin, lanolin, cholesterol, etc.) or the excipients normally used to obtain pharmaceutical hydrogels (poly(hydroxyethylmethacrylate), poly(N-vinylpyrrolidone), polyvinyl alcohol, acrylic acid polymers, such as Carbopol.)

10. A formulation, according to claim 1, characterized in that the excipient is selected among the agents that allow some of the components of the formulation to be microencapsulated, such as lipid substances (coconout oil, ethoxylated oleic glycerides, diethylene glycol monoethyl ether, C₈-C₁₀ ethoxylated glycerides, phospholipids, natural oils or petroleum derivatives or a mixture of several of them) and biocompatible polymers (polyacrylic, polylactic, polyglycolic derivatives, a polylactic-glycolic copolymer, a polyanhydride, a polyamide, a poly(alpha-amino acid), cellulosic polymers, natural polymers or a mixture of two or more of the cited compounds.)

11. Application of the formulations of the above claims 1 to 10 for the treatment of eye inflammations and/or infections.

12. Application of the formulations of the above claims 1 to 10 for the treatment of ear inflammations and/or infections.
